# EUROPÄISCHE PATENTSCHRIFT

(11) **EP 0 954 247 B1**
(45) Veröffentlichungstag und Bekanntmachung des Hinweises auf die Patenterteilung: **23.11.2005**
(21) Anmeldenummer: 97900190.6
(22) Anmeldetag: 22.01.1997
(51) Int. Cl.: A61B 17/70

(54) **VORRICHTUNG ZUR VERBINDUNG EINES LANGSTRÄGERS MIT EINER PEDIKELSCHRAUBE**
DEVICE FOR CONNECTING A LONGITUDINAL BAR TO A PEDICLE SCREW
DISPOSITIF POUR CONNECTER UN SUPPORT LONGITUDINAL AVEC UNE VIS PEDICULAIRE

(43) Veröffentlichungstag der Anmeldung: 10.11.1999
(73) Patentinhaber: Synthes AG Chur, 7002 Chur (CH)
(72) Erfinder: AMREIN, Thomas, CH-6048 Horw (CH); HESS, Martin, CH-4434 Hölstein (CH)
(74) Vertreter: Lusuardi, Werther Giovanni
(86) Internationale Anmeldenummer: PCT/CH1997/000019
(87) Internationale Veröffentlichungsnummer: WO 1998/032386

(56) Entgegenhaltungen:
- EP-A- 0 242 708
- DE-C- 19 509 332
- US-A- 5 443 467
- US-A- 5 549 608

## Beschreibung

Die Erfindung betrifft eine Vorrichtung gemäss dem Oberbegriff des Patentanspruchs 1, wie sie aus der US-A-5549608 aus bekannt hervorgeht.

Aus dem Stand der Technik sind bereits eine ganze Anzahl von Pedikelschrauben für die Wirbelsäulenfixation bekannt, welche den Vorteil besitzen, dass die einzelnen Pedikelschrauben jederzeit am Längsträger befestigt oder wieder entfernt werden können, ohne dass das gesamte Fixationssystem demontiert werden muss. Eine solche Pedikelschraube ist beispielsweise aus der EP-B 0 330 881 SHERMAN bekannt.

Der Nachteil der bekannten Pedikelschrauben besteht in den relativ komplizierten Verschlussmechanismen um den, in den offen ausgebildeten Pedikelschraubenkopf eingelegten Längsträger zu fixieren. Zudem lässt sich in den meisten Fällen der Kopf der Schraube nur bedingt zur Lage des Längsträgers ausrichten, was ein aufwendiges Zurechtbiegen des Längsträgers bedingt.

Hier will die Erfindung Abhilfe schaffen. Der Erfindung liegt das Problem zugrunde, eine Vorrichtung zur Verbindung eines Längsträgers mit einer Pedikelschraube zu schaffen, welche einfach zu handhaben ist und eine Abwinkelung der Pedikelschraube innerhalb eines gewissen Bereichs zulässt.

Die Erfindung löst die gestellte Aufgabe mit einer Vorrichtung, welche die Merkmale des Anspruchs 1 aufweist.

In einer Ausführungsart lässt sich die erfindungsgemässe Vorrichtung in der gewünschten Anzahl auf einen Längsträger aufreihen und kann dann einfach auf die bereits in den Wirbelkörpern implantierten Pedikelschrauben mit Kugelkopf aufgeklinkt werden, so dass eine primäre Verbindung zwischen Längsträger und Pedikelschraube hergestellt wird. Andere Ausführungsarten der erfindungsgemässen Vorrichtung erlauben ein nachträgliches Einlegen des Längsträgers von der Seite oder von oben. Durch das Einschrauben der Stellschraube in die Fixationsvorrichtung wird gleichzeitig der Längsträger in der Vorrichtung axial und relativ blockiert und die Vorrichtung winkelstabil fixiert. Dabei drückt die Stellschraube auf den in die Vorrichtung eingeführten Längsträger, dieser drückt auf die Hülse der Fixationsvorrichtung und die Hülse verspannt über vorzugsweise konisch ausgebildete, korrespondierende Innenflächen der Hülse und Aussenflächen der Spannzange die Zangen auf dem vorzugsweise kugeligen Kopf der Pedikelschraube. Wiederum andere Ausführungsarten erlauben ein sequentielles und unabhängiges Blockieren des Längsträgers und der Rotation der Vorrichtung in Bezug auf die Pedikelschraube.

Die erfindungsgemässe Vorrichtung bietet somit gegenüber bekannten Vorrichtungen den Vorteil, dass die Pedikelschrauben nicht nur genau senkrecht zum Längsträger fixierbar sind, sondern eine Abwinkelung von bis zu ± 25° gestatten. Dies ist besonders wichtig, wenn der Längsträger ungenau angebogen wurde, was bei herkömmlichen Systemen zu grossen Schwierigkeiten bei der Montage führt.

Eine bevorzugte Weiterbildung der erfindungsgemässen Vorrichtung besteht darin dass sie zusätzlich eine Pedikelschraube mit einem vorzugsweise kugeligen Kopf umfasst. Der Kopf der Pedikelschraube ist vorzugsweise mit einer Strukturierung in Form von Querrillen oder Querrippen versehen, um eine bessere Fixation (Verspannung gegen die Spannzange) zu erzielen. Um die Pedikelschrauben in den Knochen eindrehen zu können, sind sie im Kugelkopf vorzugsweise mit einem Innensechskant versehen.

Die Erfindung und Weiterbildungen der Erfindung werden im folgenden anhand von Ausführungsbeispielen noch näher erläutert, wobei die Figuren 1-6 und 8-14 nicht den Gegenstand der Erfindung darstellen.

Es zeigen:
Fig. 1 eine perspektivische Explosionsdarstellung einer Vorrichtung zur Verbindung eines Längsträger (1) mit einer Pedikelschraube (2) innerhalb eines Wirbelsäulenfixationssystems;
Fig. 2 einen geschlitzten Längsschnitt durch die Vorrichtung nach Fig. 1 in der Zeichenebene ;
Fig. 3 eine perspektivische Ansicht der Vorrichtung nach Fig. 1 im montierten Zustand nach Blockierung des Längsträgers und der Pedikelschraube durch die Stellschraube;
Fig. 4 eine perspektivische Ansicht einer Variante der in Fig. 1 gezeigten Vorrichtung mit Stellmutter und geteilter Hülse;
Fig. 5 eine perspektivische Ansicht einer Variante der in Fig. 4 gezeigten Vorrichtung mit nach oben offenem Kanal an der Spannzange und einem Sicherungszapfen;
Fig. 6 eine perspektivische Ansicht einer Variante der in Fig. 1 gezeigten Vorrichtung mit einer Stellmutter zur Fixierung des Kugelkopfes der Pedikelschraube und einer Stellschraube zur Blockierung des Längsträgers;
Fig. 7 einen Längsschnitt durch eine Variante der in Fig. 6 gezeigten Vorrichtung mit einer Stellschraube integriert in einer Spannschraube;
Fig. 8 eine perspektivische Ansicht einer Variante der in Fig. 5 gezeigten Vorrichtung mit einer zur Aufnahme des Längsträgers seitlich offenen Spannzange und einer Stellmutter als Spannmittel zur gleichzeitigen Blockierung des Längsträgers und Fixierung des Kugelkopfes der Pedikelschraube;
Fig. 9 eine perspektivische Ansicht der in Fig. 5 gezeigten Vorrichtung ohne Sicherungszapfen;
Fig. 10 eine perspektivische Ansicht einer Variante der in Fig. 5 gezeigten Vorrichtung mit einer Stellschraube und einem Innengewinde in der Spannzange;
Fig. 11 eine perspektivische Ansicht einer Variante der in Fig. 8 gezeigten Vorrichtung mit einer Stellmutter zur Fixierung des Kugelkopfes der Pedikelschraube und einer Stellschraube zur Blockierung des Längsträgers;
Fig. 12 eine perspektivische Ansicht einer Variante der in Fig. 11 gezeigten Vorrichtung mit einer nach oben offenen Spannzange;
Fig. 13 eine perspektivische Ansicht einer Variante der in Fig. 11 gezeigten Vorrichtung mit einer seitlich offenen Durchgangsöffnung in der Hülse; und
Fig. 14 eine perspektivische Ansicht einer Variante der in Fig. 12 gezeigten Vorrichtung mit einer nach oben offenen Durchgangsöffnung in der Hülse.

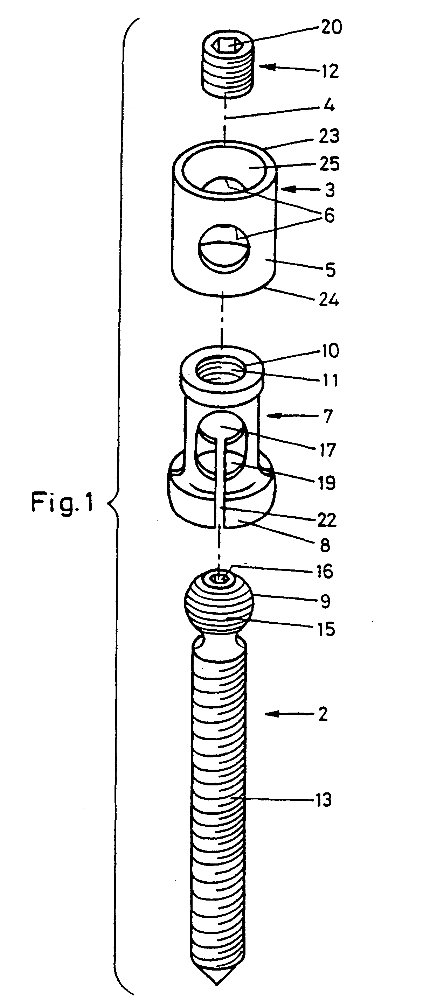

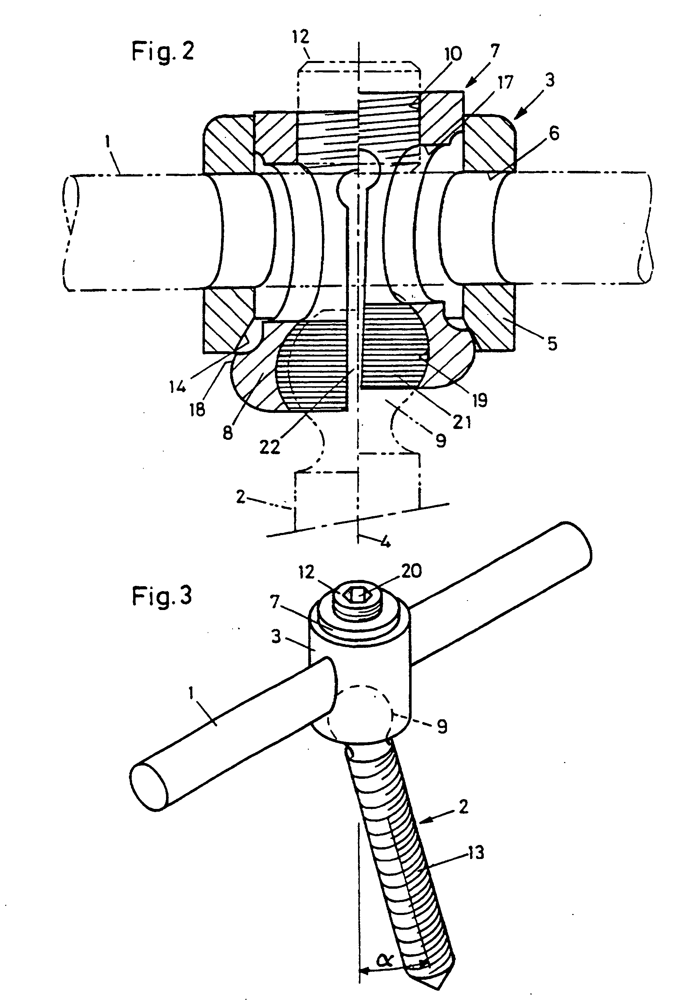

Die in Fig. 1 und 2 dargestellte Vorrichtung besteht im wesentlichen aus einem Körper 3 in Form einer hohlzylindrischen Hülse mit der Achse 4 (Achse des Hohlzylinders) und einer koaxial im Inneren des Körpers 3 gleitbar angeordneten hohlzylindrischen Spannzange 7.

Der Körper 3 besitzt eine quer zur Achse 4 verlaufende Durchgangsöffnung 6 - welche die Mantelfläche des Hohlzylinders symmetrisch zur Achse 4 an zwei Stellen durchstösst - und auch die Spannzange 7 besitzt eine quer zur Achse 4 verlaufende Durchgangsöffnung 17. Durch eine fluchtende Anordnungen der Durchgangsöffnungen 6 und 17 wird die Einführung eines quer zur Achse 4 verlaufenden Längsträgers 1 ermöglicht, wie dies in Fig. 2 dargestellt ist.

Die Spannzange 7 ist in ihrem oberen Teil innerhalb des (hohlzylindrischen) Körpers 3 durch Presssitz befestigt und lässt sich bei Überwindung der relativ hohen Kraft des Presssitzes axial gegenüber dem Körper 3 verschieben. Zweckmässigerweise ist der (hohlzylindrische) Körper 3 und die Spannzange 7 gegeneinander verdrehsicher angeordnet, z.B. mittels entsprechender axial verlaufender Nuten/Rillen-Führungen. Die Spannzange 7 ist mit nach unten gerichteten, gegen die Achse 4 federnd ausgebildeten Zungen 8 ausgebildet, in welche von unten der kugelige Kopf 9 einer Pedikelschraube 2 federnd einschnappbar ist.

Im oberen Teil der Spannzange 7 ist eine Bohrung 10 mit Innengewinde 11 vorgesehen, zur Aufnahme einer Stellschraube 12 mit Innensechskant 20, welche auf einen in die Vorrichtung eingeführten Längsträger 1 eine Kraft ausüben kann, wodurch der Längsträger 1 gegenüber dem Körper 3 axial und rotativ blockierbar ist, wie dies in Fig. 3 angedeutet ist. Die Spannzange 7 ist an ihrem unteren Ende derart ausgebildet, dass der am kugeligen Kopf 9 anschliessende Schraubenschaft 13 der Pedikelschraube 2 in einem Winkel Ó von - 25° bis + 25° gegenüber der Achse 4 blockierbar ist.

Der Körper 3 weist an seinem unteren Ende 24 eine sich nach unten erweiternde konische Innenfläche 14 auf, gegen welche eine entsprechend ausgebildete konische Aussenfläche 18 der freien Enden der Zangen 8 der Spannzange 7 gleitend zur Anlage bringbar ist. Sobald die Stellschraube 12 auf den in die Vorrichtung eingeführten Längsträger 1 stösst, beginnt sich die Spannzange 7 gegenüber dem Körper 3 nach oben zu schieben. Dieser Vorgang ist in Fig. 2 dargestellt, wo in der linken Hälfte der Zustand vor dem Hochschieben und in der rechten Hälfte das Aneinander gleiten der konischen Flächen 14 und 18 beim Hochschieben der Spannzange 7 dargestellt ist, wobei die durch die Schlitze 22 federnd ausgebildeten Zangen 8 der Spannzange 7 zur Zylinderachse 4 hin gedrückt werden, so dass die hohlkugelförmige Kavität 19 verkleinert wird und den darin eingeführten Kopf 9 der Pedikelschraube 2 fest umklammert. Um die Blockierung des Kopfes 9 zu optimieren ist dieser mit einer Strukturierung 15, vorzugsweise in Form von Querrillen, bzw. Querrippen versehen. Die Pedikelschraube 2 entspricht im übrigen den bekannten Schrauben-Konstruktionen und ist zweckmässigerweise mit einem Innensechskant 16 versehen.

Die hohlkugelförmige Kavität 19 kann ihrerseits mit einer Strukturierung 21, vorzugsweise in Form von Querrillen, bzw. Querrippen versehen sein. Auch eine Kombination der Strukturierungen 21 und 15 ist möglich um die Fixierung des Kopfes 9 in der hohlkugelförmige Kavität 19 weiter zu verbessern.
Die hohlkugelförmige Kavität 19 ist zweckmässigerweise komplementär zur Form des aufzunehmenden Kopfes 9 der Pedikelschraube 2 ausgebildet.

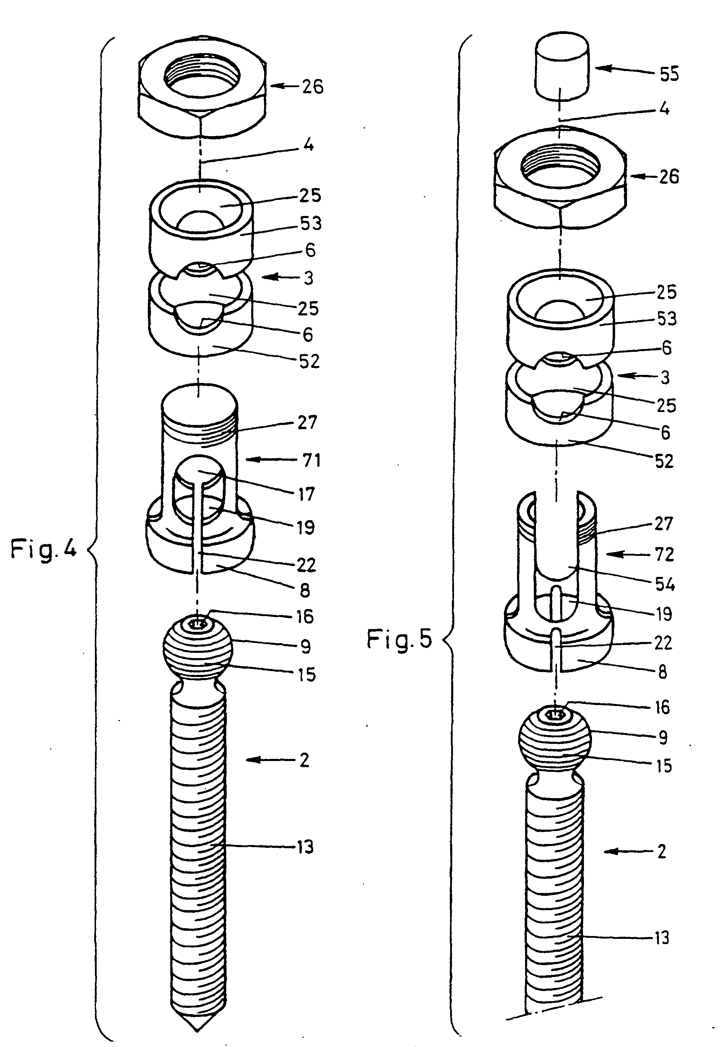

Die in Fig. 4 dargestellte Variante der Vorrichtung zeigt die Anwendung eines quer zur Achse 4 in der Mitte der Durchgangsöffnung 6 in ein Unterteil 52 und ein Oberteil 53 getrennten Körpers 3 in Verbindung mit einer Stellmutter 26. Der Unterteil 52 des Körpers 3 muss so hoch sein, dass der Längsträger 1 immer auf dem Unterteil 52 aufliegt. Die Spannzange 71 unterscheidet sich von der in Fig. 1 abgebildeten Spannzange 7 nur dadurch, dass am oberen Ende das Innengewinde 11 durch ein Aussengewinde 27 zum Gebrauch einer Stellmutter 26 ersetzt worden ist. Die Stellmutter 26 drückt, wenn sie angezogen wird, auf das Oberteil 53 des Körpers 3, welches in der Folge direkt auf den Längsträger 1 drückt. Daraufhin wird der Längsträger 1 auf das Unterteil 52 des Körpers 3 gepresst und dieses blockiert dann wie in Fig. 2 gezeigt den Kugelkopf 9.

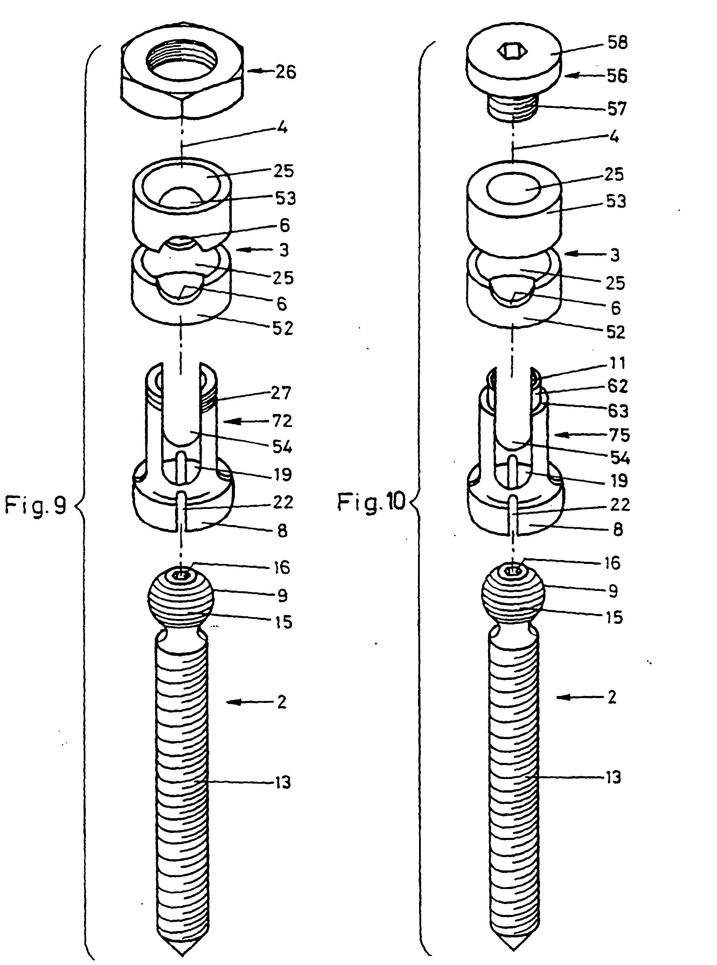

Die in Fig. 5 dargestellte Variante der Vorrichtung unterscheidet sich von der in Fig. 9 abgebildeten Variante nur dadurch, dass ein Zapfen 55 das Kollabieren der durch den Kanal 54 geschwächten Spannzange 72 beim Anziehen der Stellmutter 26 verhindert. Um ein Herausfallen des Zapfens 55 zu vermeiden, wird dieser mit Vorteil mit einem Aussengewinde versehen und eingeschraubt.

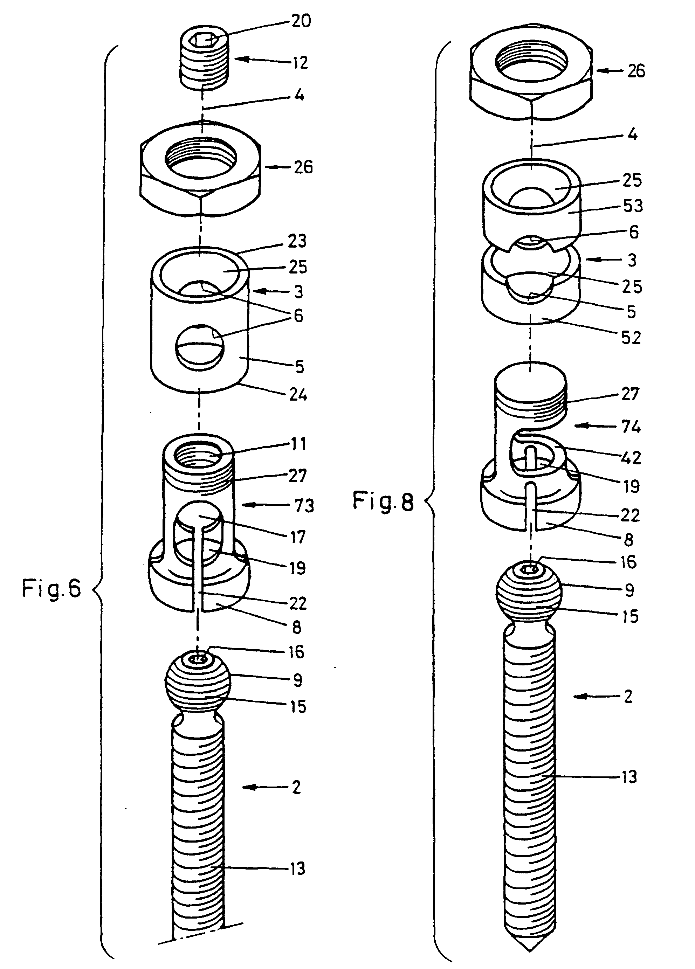

Die in Fig. 6 dargestellte Variante der Vorrichtung unterscheidet sich von der in Fig. 4 abgebildeten Variante nur dadurch, dass
a) die Spannzange 73 neben dem Aussengewinde 27 noch ein Innengewinde 11 aufweist und
b) der Körper 3 einteilig und die Durchgangsöffnung 6 gegenüber der Durchgangsöffnung 17 derart plaziert ist, dass beim Anziehen der Stellmutter 26 nur der Kugelkopf 9 blockiert wird. Dies ermöglicht eine winkelstabile Distraktion bzw. Kompression. Die Stellmutter 26 drückt auf den Körper 3 und die damit erreichte Verspannung des Körpers 3 mit der Spannzange 73 fixiert den Kugelkopf 9 der Pedikelschraube 2 ohne den Längsträger 1 zu blockieren. Der Längsträger 1 wird separat mit Hilfe der Stellschraube 12 auf den unteren Rand der Durchgangsöffnung 6 des Körpers 3 gedrückt. Dadurch wird der Körper 3 noch weiter mit der Spannzange 73 verkeilt, was zu einer verstärkten Klemmung des Kugelkopfes 9 führt. Die Durchgangsöffnung 6 muss im Körper 3 so plaziert sein, dass der Längsträger 1 auch nach dem Anziehen der Stellmutter 26 auf dem unteren Rand der Durchgangsöffnung 6 aufliegt.

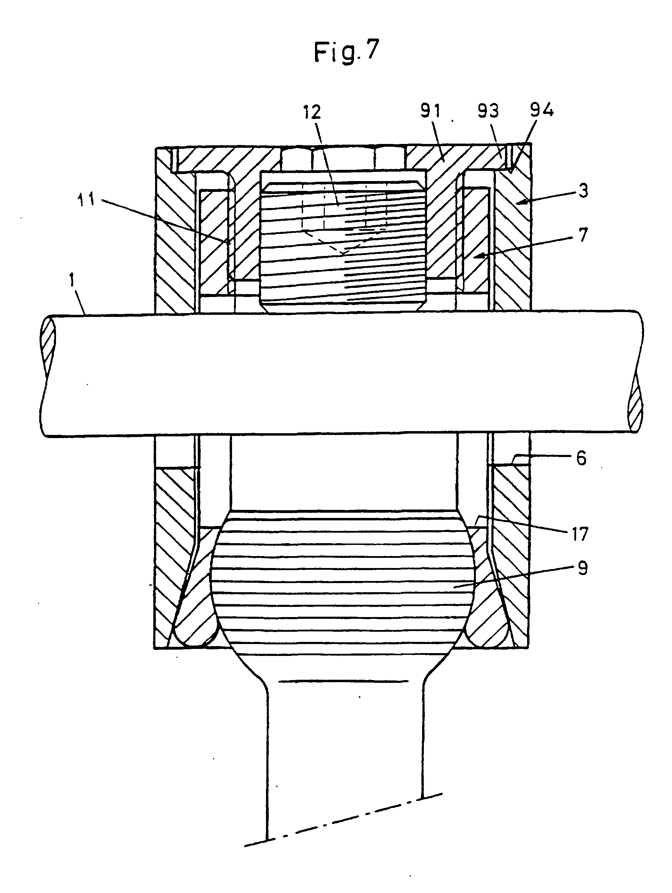

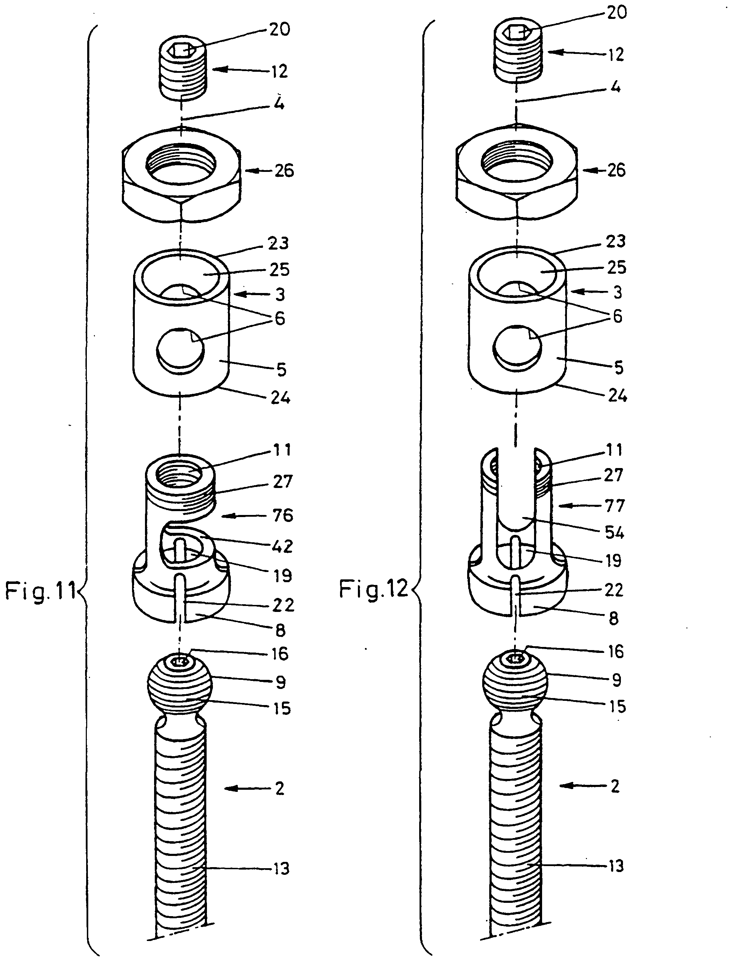

Die in Fig. 7 dargestellte Variante der erfindungsgemässen Vorrichtung unterscheidet sich nur durch die Ausführung der Spannschraube 91 und der Positionierung der Durchgangsöffnung 6 gegenüber der Durchgangsöffnung 17 von der in Fig. 1 bis 3 gezeigten Vorrichtung. Die Spannzange 7 ist mit einem Innengewinde 11 versehen. Zur Blockierung des Kugelkopfes 9 und des Längsträgers 1 dienen eine Spannschraube 91, die in das Innengewinde 11 eingedreht wird, und eine Stellschraube 12, die in die Spannschraube 91 integriert ist. Der Körper 3 ist analog zu Fig. 1-3 einteilig und oben mit einer Andrehung 94 versehen, die Schulter 93 der Spannschraube 91 aufnimmt. Die Durchgangsöffnung 6 im Körper 3 ist gegenüber der Durchgangsöffnung 17 in der Spannzange 7 derart positioniert, dass beim Anziehen der Spannschraube 91 nur der Kugelkopf 9 blockiert wird. Der Längsträger 1 wird mittels der Stellschraube 12 nachträglich fixiert. Solange die Stellschraube 12 vollständig in die Spannschraube 91 eingedreht ist und die Spannschraube 91 soweit zurückgedreht ist, dass der Längsträger 1 den unteren Rand der Durchgangsöffnung 6 im Körper 3 berührt, kann der Kugelkopf 9 beliebig ein- und ausgehängt werden. Auch wird beim Zurückdrehen der Spannschraube 91 aus dem festgezogenen Zustand die Spannzange 7 wieder freigestellt. Die Spannzange 7 kann zur Aufnahme des Längsträgers 1 mit einer Durchgangsöffnung 17 gemäss Fig. 4, mit einem noch oben offenen Kanal 54 gemäss Fig. 9 oder mit einem seitlich offenen Kanal 42 gemäss Fig. 8 versehen sein. In diesen beiden Fällen ist entweder eine zweiteilige Ausführung des Körpers 3 oder eine Ausführung gemäss Fig. 11 und 12 von Vorteil, um das Einführen des Längsträgers zu erleichtern. Bei der zweiteiligen Ausführung müssen die beiden Teile des Körpers 3 aber dabei derart ausgelegt sein, dass der obere Teil 53 auch nach dem Verspannen direkt auf den unteren Teil 52 und nicht auf den Längsträger 1 drückt.

Die in Fig. 8 dargestellte Variante der Vorrichtung unterscheidet sich von der in Fig. 4 abgebildeten Variante nur dadurch, dass im Gegensatz zur Spannzange 71 die Spannzange 74 zur Aufnahme des Längsträgers 1 anstelle der Durchgangsöffnung 17 einen seitlich offenen Kanal 42 aufweist, wobei die Schlitze 22 nicht in diesen hineinreichen.

Die in Fig. 9 dargestellte Variante der Vorrichtung unterscheidet sich von der in Fig. 4 abgebildeten Variante nur dadurch, dass im Gegensatz zur Spannzange 71 die Spannzange 72 zur Aufnahme des Längsträgers 1 anstelle der Durchgangsöffnung 17 einen nach oben offenen Kanal 54 aufweist, wobei die Schlitze 22 nicht in diesen hineinreichen. Um ein Kollabieren der Spannzange 72 beim Anziehen der Stellmutter 26 zu verhindern, muss das Gewinde derart konzipiert werden, dass beim Anziehen vernachlässigbar kleine radiale Kräfte auftreten. Ein Sägezahngewinde würde zum Beispiel dieser Anforderung genügen.

Die in Fig. 10 dargestellte Variante der Vorrichtung zeigt die Anwendung einer Spannzange 75, die im Gegensatz zu der in Fig. 5 und 9 abgebildeten Spannzange 72 anstelle des Aussengewindes 27 mit einem Innengewinde 11 versehen ist. Als Spannmittel dient eine Stellschraube 56 mit einem Aussengewinde 57 und einem Bord 58. Die Stellschraube 56 drückt beim Anziehen auf den Längsträger 1, der seinerseits auf das Unterteil 52 des Körpers 3 drückt. Dieses Verspannen von Unterteil 52 und Spannzange 75 blockiert wie in Fig. 2 gezeigt den Kugelkopf 9 der Pedikelschraube 2. Der Oberteil 53 des Körpers 3 verhindert beim Anziehen der Stellschraube 56 ein Aufweiten der Spannzange 75. Der Oberteil 53 steht an der Schulter 63 auf.

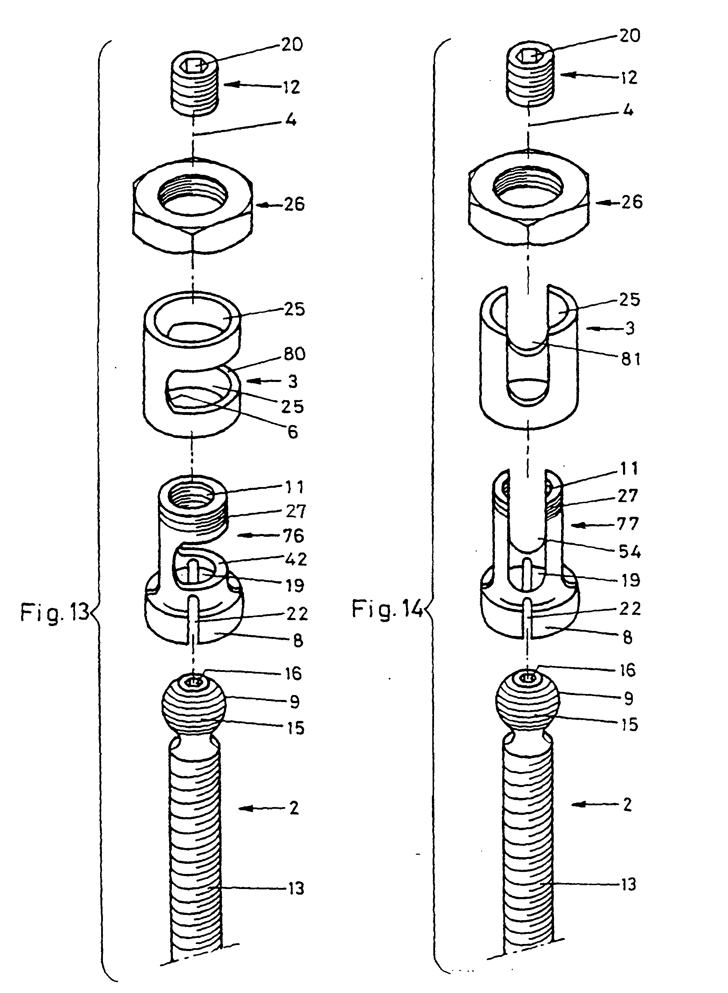

Die in Fig. 11 dargestellte Variante der Vorrichtung unterscheidet sich von der in Fig. 6 abgebildeten Variante nur dadurch, dass im Gegensatz zur Spannzange 73 die Spannzange 76 zur Aufnahme des Längsträgers 1 anstelle der Durchgangsöffnung 17 einen seitlich offenen Kanal 42 aufweist und die Schlitze 22 nicht in diesen hineinreichen. Für ein ungehindertes Einführen des Längsträgers 1 ist entsprechend Fig. 13 die Durchgangsöffnung 6 im Körper 3 mit Vorteil seitlich offen (80).

Die in Fig. 12 dargestellte Variante der Vorrichtung unterscheidet sich von der in Fig. 11 abgebildeten Variante nur dadurch, dass im Gegensatz zur Spannzange 76 die Spannzange 77 zur Aufnahme des Längsträgers 1 einen nach oben offenen Kanal 54 aufweist. Für ein ungehindertes Einführen des Längsträgers 1 ist entsprechend Fig. 14 die Durchgangsöffnung 6 im Körper 3 mit Vorteil nach oben offen (81).

Die in Fig. 13 dargestellte Variante der Vorrichtung unterscheidet sich von der in Fig. 11 abgebildeten Variante nur dadurch, dass im Körper 3 eine seitlich offene Durchgangsöffnung 80 angebracht ist.

Die in Fig. 14 dargestellte Variante der Vorrichtung unterscheidet sich von der in Fig. 12 abgebildeten Variante nur dadurch, dass im Körper 3 eine nach oben offene Durchgangsöffnung 81 angebracht ist.

## Patentansprüche

1. Vorrichtung zur Verbindung eines Längsträgers (1) mit einer Pedikelschraube (2) innerhalb eines Wirbelsäulenfixationssystems umfassend
A) einen Körper (3) mit einem oberen Ende (23), einem unteren Ende (24), einer zumindest nach unten offenen Bohrung (25) mit der Achse (4) und einer quer zur Achse (4) durch die Bohrung (25) angebrachte Durchgangsöffnung (6), durch welche ein quer zur Achse (4) verlaufender Längsträger (1) einführbar ist;
B) eine koaxial im Innern des Körpers (3) entlang der Achse (4) gleitbar angeordnete Spannzange (7), mit einer zur Durchgangsöffnung (6) des Körpers (3) fluchtenden Durchgangsöffnung (17) und mit einer zumindest nach unten gerichteten, durch, gegen die Zylinderachse (4) federnd ausgebildeten Zungen (8), begrenzte Kammer (19), in welche von unten der Kopf (9) einer Pedikelschraube (2) federnd einschnappbar ist; und
C) ein Spannmittel (26;91) zur Bewegung der Spannzange (7) relativ zum Körper (3),
**dadurch gekennzeichnet, dass**
D) die Durchgangsöffnung (6) im Körper (3) gegenüber der Durchgangsöffnung (17) in der Spannzange (7) derart plaziert ist, dass beim Anziehen des Spannmittels (26;91) nur der Kopf einer Pedikelschraube (2) blockiert wird; und
E) eine Stellschraube (12) im Spannmittel (26;91) derart integriert ist, dass sie beim Runterdrehen einen in die Vorrichtung eingeführten Längsträger (1) blockiert.

2. Vorrichtung nach Anspruch 1, **dadurch gekennzeichnet, dass** die Kammer (19) als hohlkugelförmige Kavität ausgebildet ist.

3. Vorrichtung nach Anspruch 2, **dadurch gekennzeichnet, dass** die Spannzange (7) aussen im Bereich der als hohlkugelförmige Kavität ausgebildeten Kammer (19) gegen unten sich erweiternd, konisch ausgebildet ist.

4. Vorrichtung nach einem der Ansprüche 1 bis 3, **dadurch gekennzeichnet, dass** eine Stellschraube (12) in der Spannzange (7) oder in einem Spannmittel (91) so integriert ist, dass sie beim Runterdrehen den Längsträger (1) blockiert.

5. Vorrichtung nach einem der Ansprüche 1 bis 4, **dadurch gekennzeichnet, dass** die Spannzange (7) symmetrisch zu einer Symmetrieebene ist.

6. Vorrichtung nach einem der Ansprüche 1 bis 5, **dadurch gekennzeichnet, dass** der Körper (3) symmetrisch zu einer Symmetrieebene ist.

7. Vorrichtung nach einem der Ansprüche 1 bis 6, **dadurch gekennzeichnet, dass** der Körper (3) in einen oberen Teil (53) und einen unteren Teil (52) getrennt ist, wobei die Trennfläche quer zur Achse (4) im Bereich der Durchgangsöffnung (6) verläuft und die Bohrung (25) zumindest im unteren Teil (52) durchgehend ausgebildet ist.

8. Vorrichtung nach einem der Ansprüche 1 bis 7, **dadurch gekennzeichnet, dass** das Spannmittel (12;56) auf den Längsträger (1) drückt und **dadurch** die Spannzange (7) in den Körper (3) zieht und damit ein Festklemmung des Kopfes (9) der Pedikelschraube (2) und des Längsträgers (1) bewirkt.

9. Vorrichtung nach einem der Ansprüche 1 bis 8, **dadurch gekennzeichnet, dass** die Höhe der Durchgangsöffnung (6) derart bemessen ist, dass in jeder Position des Spannmittels (12;26;56) der Längsträger (1) immer auf dem unteren Rand der Durchgangsöffnung (6) aufliegt.

10. Vorrichtung nach einem der Ansprüche 1 bis 9, **dadurch gekennzeichnet, dass** der Körper (3) und die Spannzange (7) gegeneinander verdrehsicher aber axial verschiebbar angeordnet sind.

11. Vorrichtung nach einem der Ansprüche 1 bis 10, **dadurch gekennzeichnet, dass** die Spannzange (7) mittels Pressitz im Körper (3) angeordnet ist.

12. Vorrichtung nach einem der Ansprüche 1 bis 11, **dadurch gekennzeichnet, dass** der Körper (3) an seinem unteren Ende (24) eine sich nach unten erweiternde konische Innenfläche (14) aufweist, gegen welche eine entsprechend ausgebildet konische Aussenfläche (18) der freien Enden der Zungen (8) der Spannzange (7) gleitend zur Anlage bringbar ist.

13. Vorrichtung nach einem der Ansprüche 1 bis 12, **dadurch gekennzeichnet, dass** die Spannzange (7) im Kontaktbereich mit dem Körper (3) derart beschaffen ist, dass sie tangential auf der nach unten erweiternden konischen Innenfläche (14) des Körpers (3) aufliegt.

14. Vorrichtung nach einem der Ansprüche 1 bis 13, **dadurch gekennzeichnet, dass** sie zusätzlich eine Pedikelschraube (2) mit einem vorzugsweise kugeligen Kopf (9) umfasst.

15. Vorrichtung nach Anspruch 14, **dadurch gekennzeichnet, dass** der Kopf (9) der Pedikelschraube (2) mit einer Strukturierung (15), vorzugsweise in Form von Querrillen, bzw. Querrippen versehen ist.

16. Vorrichtung nach Anspruch 14 oder 15, **dadurch gekennzeichnet, dass** der Kopf (9) der Pedikelschraube (2) mit einem Innensechskant (16) versehen ist.

17. Vorrichtung nach einem der Ansprüche 1 bis 16, **dadurch gekennzeichnet, dass** die Kammer (19) als hohlkugelförmige Kavität ausgebildet ist, vorzugsweise mit einer komplementären Form zur Form des aufzunehmenden Kopfes (9) der Pedikelschraube (2).

18. Vorrichtung nach einem der Ansprüche 1 bis 17, **dadurch gekennzeichnet, dass** die Kammer (19) mit einer Strukturierung (21), vorzugsweise in Form von Querrillen, bzw. Querrippen versehen ist.

19. Vorrichtung nach einem der Ansprüche 1 bis 18, **dadurch gekennzeichnet, dass** der Körper (3) als hohlzylindrische Hülse ausgebildet ist.

20. Vorrichtung gemäss Anspruch 19, **dadurch gekennzeichnet, dass** der Körper (3) einteilig ist.

21. Vorrichtung gemäss Anspruch 19, **dadurch gekennzeichnet, dass** der Körper (3) aus einem Unterteil (52) und einem Oberteil (53) besteht.

22. Vorrichtung gemäss Anspruch 21, **dadurch gekennzeichnet, dass** die Trennung zwischen dem Unterteil (52) und dem Oberteil (53) im Bereich der Durchgangsöffnung (6) stattfindet.

23. Vorrichtung nach einem der Ansprüche 1 bis 22, **dadurch gekennzeichnet, dass** die Durchgangsöffnung (6;80;81) des Körpers (3) gegenüber der Durchgangsöffnung (17;42;54) der Spannzange (7;71;72;73;74;75;76;77) derart plaziert ist, dass der Längsträger (1) auf dem unteren Rand der Durchgangsöffnung (6;80;81) des Körpers (3) aufliegt.

24. Vorrichtung nach einem der Ansprüche 1 bis 22, **dadurch gekennzeichnet, dass** die Durchgangsöffnung (6;80;81) des Körpers (3) gegenüber der Durchgangsöffnung (17;42;54) der Spannzange (7;71;72;73;74;75;76;77) derart plaziert ist, dass der Längsträger (1) auf dem unteren Rand der Durchgangsöffnung (17;42;54) der Spannzange (7) aufliegt.

25. Vorrichtung nach einem der Ansprüche 21 bis 24, **dadurch gekennzeichnet, dass** der Oberteil (53) des Körpers (3) auf dem Längsträger (1) aufliegt.

26. Vorrichtung nach einem der Ansprüche 20 bis 24, **dadurch gekennzeichnet, dass** der obere Rand der Durchgangsöffnung (6;80;81) des Körpers (3) nicht auf den Längsträger zu liegen kommt.

27. Vorrichtung nach einem der Ansprüche 1 bis 25, **dadurch gekennzeichnet, dass** an der Spannzange (74;76) zur Aufnahme des Längsträgers (1) ein seitlich offener Kanal (42) angebracht ist.

28. Vorrichtung nach einem der Ansprüche 1 bis 25, **dadurch gekennzeichnet, dass** an der Spannzange (72;75;77) zur Aufnahme des Längsträgers (1) ein nach oben offener Kanal (54) angebracht ist.

29. Vorrichtung nach einem der Ansprüche 1 bis 28, **dadurch gekennzeichnet, dass** die Spannzange (7;75) im oberen Ende mit einem Innengewinde (11) versehen ist.

30. Vorrichtung nach Anspruch 29, **dadurch gekennzeichnet, dass** als Spannmittel eine Stellschraube (56) mit einem Aussengewinde (57) eingesetzt wird.

31. Vorrichtung nach Anspruch 30, **dadurch gekennzeichnet, dass** die Stellschraube (56) mit einem Bord (58) mit einem Aussendurchmesser vorzugsweise grösser als der Innendurchmesser des Körpers (3) versehen ist.

32. Vorrichtung nach Anspruch 29, **dadurch gekennzeichnet, dass** die Durchgangsöffnung (6;80;81) im Körper (3) gegenüber der Durchgangsöffnung (17;42;54) in der Spannzange (7) derart plaziert ist, dass beim Anziehen des Spannmittels (91) nur der Kugelkopf (9) blockiert wird.

33. Vorrichtung nach Anspruch 31 oder 32, **dadurch gekennzeichnet, dass** der Körper (3) einteilig ist.

34. Vorrichtung nach Anspruch 31 oder 32, **dadurch gekennzeichnet, dass** der Körper (3) aus einem Unterteil (52) und einem Oberteil (53) besteht.

35. Vorrichtung nach einem der Ansprüche 31 bis 34, **dadurch gekennzeichnet, dass** die Durchgangsöffnung (6;80;81) im Körper (3) gegenüber der Durchgangsöffnung (17;42;54) in der Spannzange (7;71;72;74;75) derart plaziert ist, dass der Längsträger (1) vor und nach dem Anziehen des Spannmittels (26;91) auf dem unteren Rand der Durchgangsöffnung (6;80;81) aufliegt.

36. Vorrichtung nach einem der Ansprüche 31 bis 35, **dadurch gekennzeichnet, dass** die Durchgangsöffnung (6;80;81) im Körper (3) gegenüber der Durchgangsöffnung (17;42;54) in der Spannzange (7;73;76;77) derart plaziert ist, dass der Längsträger (1) auch nach dem Anziehen des Spannmittels (26;91) auf dem unteren Rand der Durchgangsöffnung (17;42;54) in der Spannzange (7;73;76;77) aufliegt.

37. Vorrichtung nach einem der Ansprüche 31 bis 36, **dadurch gekennzeichnet, dass** die Durchgangsöffnung (80) seitlich offen ist.

38. Vorrichtung nach einem der Ansprüche 31 bis 36, **dadurch gekennzeichnet, dass** die Durchgangsöffnung (81) nach oben offen ist.

39. Vorrichtung nach einem der Ansprüche 31 bis 38, **dadurch gekennzeichnet, dass** der Körper (3) derart zug- und druckfest mit dem Spannmittel (26;91) verbunden ist, vorzugsweise über eine Einkerbung (94), dass das Spannmittel (26;91) ungehindert ein- und zurückgedreht werden kann und dass beim Zurückdrehen des Spannmittels (26;91) der Körper (3) zurückgezogen und somit die Spannzange (7;73;76;77) wieder freigestellt wird.

40. Vorrichtung nach einem der Ansprüche 31 bis 38, **dadurch gekennzeichnet, dass** die Spannzange (7;73;76;77) im oberen Ende mit einem Innengewinde (11) versehen ist, in das eine Stellschraube (12) eingedreht werden kann, um den Längsträger (1) separat zu blockieren.

41. Vorrichtung nach Anspruch 36 und einem der Ansprüche 31 bis 38, **dadurch gekennzeichnet, dass** die Durchgangsöffnung (6) derart geformt ist, dass der Oberteil (53) nicht auf den Längsträger (1) zu liegen kommt.

42. Vorrichtung nach einem der Ansprüche 1 bis 41, **dadurch gekennzeichnet, dass** die Bohrung (25) im Körper (3) durchgehend von unten nach oben ausgebildet ist.

## Claims

1. Device for connecting a longitudinal bar (1) to a pedicle screw (2) within a system for fixation of the spinal column, comprising
A) a one-piece body (3) with an upper end (23), a lower end (24), a hole (25) which is open at least at the bottom and has the axis (4) and a through hole (6) provided through the hole (25) and runs across the axis (4) so that a longitudinal bar (1) running across this axis (4) can be inserted through this through hole; and
B) a collet chuck (7) arranged coaxially in the interior of the body (3) so that it can slide along the axis (4), with a through hole (17) which is aligned with the through hole (6) of the body (3), and with a chamber (19) which is pointing downward at least and is bordered by tongues (8) which are designed to have a spring action against the cylinder axis (4), so that the head (9) of a pedicle screw (2) can be snapped into this chamber from below with a spring action; and
C) a tensioning means (26;91) is provided which can move the collet chuck (7) relative to the body (3),
**characterised in that**
D) the through hole (6) in the body (3) is positioned with respect to the through hole (17) in the collet chuck (7) such that only the spherical head (9) is locked when the tensioning means (26;91) is tightened; and
E) a set screw (12) is integrated into the tensioning means (26; 91) in such a way that it locks the longitudinal bar (1) when tightened down.

2. Device according to claim 1, **characterized in that** the chamber (19) is designed as a hollow spherical cavity.

3. Device according to claim 2, **characterized in that** the collet chuck (7) is conical in design on the outside, becoming wider toward the bottom, in the area of the chamber (19) which is designed as a hollow spherical cavity.

4. Device according to one of claims 1 through 3, **characterized in that** a set screw (12) is integrated into the collet chuck (7) or into a tensioning means (91) so that it locks the longitudinal bar (1) when screwed down.

5. Device according to one of claims 1 through 4, **characterized in that** the collet chuck (7) is designed symmetrical to a plane of symmetry.

6. Device according to one of claims 1 through 5, **characterized in that** the body (3) is designed symmetrical to a plane of symmetry.

7. Device according to one of claims 1 through 6, **characterized in that** the body (3) is divided into a top part (53) and a bottom part (52), with the dividing surface running across the axis (4) in the area of the through hole (6), and the hole (25) is designed to be continuous at least in the bottom part (52).

8. Device according to one of the claims 1 through 7, **characterized in that** the tensioning means (12; 56) presses on the longitudinal bar (1) and thereby pulls the collet chuck (7) into the body (3) and thus causes the head (9) of the pedicle screw (2) and the longitudinal bar (1) to be clamped securely.

9. Device according to one of claims 1 through 8, **characterized in that** the height of the through hole (6) is designed so that in any position of the tensioning means (12; 26; 56) the longitudinal bar (1) is always in contact with the lower edge of the through hole (6).

10. Device according to one of claims 1 through 9, **characterized in that** the body (3) and the collet chuck (7) are arranged so that they are rotationally locked but axially displaceable relative to one another.

11. Device according to one of claims 1 through 10, **characterized in that** the collet chuck (7) is arranged in the body (3) by means of a press fit.

12. Device according to one of claims 1 through 11, **characterized in that** at its lower end (24) the body (3) has a conical inside surface (14) which becomes wider at the bottom, and a suitably designed conical outside surface (18) of the free ends of the tongues (8) of the collet chuck (7) can be brought into sliding contact with this inside surface.

13. Device according to one of claims 1 through 12, **characterized in that** the collet chuck (7) is designed in the contact area with the body (3) so that it is in contact tangentially with the conical inside surface (14) of the body (3).

14. Device according to one of claims 1 through 13, **characterized in that** it also includes a pedicle screw (2) with a preferably spherical head (9).

15. Device according to claim 14, **characterized in that** the head (9) of the pedicle screw (2) is provided with a structuring (15), preferably in the form of transverse grooves or transverse ribs.

16. Device according to claim 14 or 15, **characterized in that** the head (9) of the pedicle screw (2) is provided with an hexagonal socket head (16).

17. Device according to one of claims 1 through 16, **characterized in that** the chamber (19) is designed as a hollow spherical cavity, preferably with a shape that is complementary to the shape of the head (9) of the pedicle screw (2) which is to be accommodated.

18. Device according to one of claims 1 through 17, **characterized in that** the chamber (19) is provided with a structuring (21), preferably in the form of transverse grooves or transverse ribs.

19. Device according to one of claims 1 through 18, **characterized in that** the body (3) is designed as a hollow cylindrical bushing.

20. Device according to claim 19, **characterized in that** the body (3) is designed in one piece.

21. Device according to claim 19, **characterized in that** the body (3) consists of a bottom part (52) and a top part (53).

22. Device according to claim 21, **characterized in that** the separation between the bottom part (52) and the top part (53) is in the area of the through hole (6).

23. Device according to one of claims 1 through 22, **characterized in that** the through hole (6; 80; 81) of the body (3) is positioned with respect to the through hole (17; 42; 54) of the collet chuck (7; 71; 72; 73; 74; 75; 76; 77) in such a way that the longitudinal bar (1) rests on the lower edge of the through hole (6; 80; 81) of the body (3).

24. Device according to one of claims 1 through 22, **characterized in that** the through hole (6; 80; 81) of the body (3) is positioned relative to the through hole (17; 42; 54) of the collet chuck (7; 71; 72; 73; 74; 75; 76; 77) so that the longitudinal bar (1) rests on the lower edge of the through hole (17; 42; 54) of the collet chuck (7).

25. Device according to one of claims 21 through 24, **characterized in that** the top part (53) of the body (3) rests on the longitudinal bar (1).

26. Device according to one of claims 20 through 24, **characterized in that** the upper edge of the through hole (6; 80; 81) of the body (3) does not come to rest against the longitudinal bar.

27. Device according to one of claims 1 through 25, **characterized in that** a channel (42) that is open at the side is provided on the collet chuck (74; 76) to accommodate the longitudinal bar (1).

28. Device according to one of claims 1 through 25, **characterized in that** a channel (54) that is open at the top is provided on the collet chuck (72; 75; 77) to accommodate the longitudinal bar (1).

29. Device according to one of claims 1 through 28, **characterized in that** the collet chuck (7;75) is provided with an outside thread (27) on the upper end.

30. Device according to claim 29, **characterized in that** a set screw (56) with an outside thread (57) is used as the tensioning means.

31. Device according to claim 30, **characterized in that** the set screw (56) is provided with a flange (58) having an outside diameter which is preferably greater than the inside diameter of the body (3).

32. Device according to claim 29, **characterized in that** the through hole (6; 80; 81) in the body (3) is positioned with respect to the through hole (17; 42; 54) in the collet chuck (7; 73; 76; 77) such that only the spherical head (9) is locked when the tensioning means (91) is tightened.

33. Device according to claim 31 or 32, **characterized in that** the body (3) is designed in one piece.

34. Device according to claim 31 or 32, **characterized in that** the body (3) consists of a bottom part (52) and a top part (53).

35. Device according to one of claims 31 through 34, **characterized in that** the through hole (6; 80; 81) is positioned in the body (3) with respect to the through hole (17; 42; 54) in the collet chuck (7; 71; 72; 74; 75) such that the longitudinal bar (1) rests on the lower edge of the through hole (6; 80; 81) before and after tightening the tensioning means (26; 91).

36. Device according to one of claims 31 through 35, **characterized in that** the through hole (6; 80; 81) is positioned in the body (3) with respect to the through hole (17; 42; 54) in the collet chuck (7; 73; 76; 77) such that the longitudinal bar (1) rests on the lower edge of the through hole (17; 42; 54) in the collet chuck (7; 73; 76; 77) even after tightening the tensioning means (26; 91).

37. Device according to one of claims 31 through 36, **characterized in that** the through hole (80) is open at the side.

38. Device according to one of claims 31 through 36, **characterized in that** the through hole (81) is open at the top.

39. Device according to one of claims 31 through 38, **characterized in that** the body (3) is connected to the tensioning means (26; 91) with tensile and compressive strength, preferably by a notch (94), in such a way that the tensioning means (26; 91) can be tightened and unscrewed, and the body (3) can be withdrawn by screwing the tensioning means (26; 91) in reverse, thus releasing the collet chuck (7; 73; 76; 77) again.

40. Device according to one of claims 31 through 38, **characterized in that** the collet chuck (7; 73; 76; 77) is provided with an inside thread (11) in the upper end into which a set screw (12) can be screwed to lock the longitudinal bar (1) separately.

41. Device according to claim 36 and one of claims 31 through 38, **characterized in that** the through hole (6) is shaped so that the top part (53) does not come to rest on the longitudinal bar (1).

42. Device according to one of claims 1 through 41, **characterized in that** the hole (25) in the body (3) is designed to be continuous from bottom to top.

## Revendications

1. Dispositif permettant de relier un support longitudinal (1) à une vis pédiculaire (2) dans un système de fixation pour colonne vertébrale, ledit dispositif comprenant :
A) un corps (3) avec une extrémité supérieure (23), une extrémité inférieure (24), un trou (25) ouvert au moins vers le bas et présentant l'axe (4) ainsi qu'un trou de passage (6) formé à travers le trou (25) de manière transversale audit axe (4) et à travers lequel peut être inséré un support longitudinal (1) s'étendant transversalement à l'axe (4);
B) une pince de serrage (7) disposée à l'intérieur du corps (3) de manière à pouvoir glisser coaxialement le long de l'axe (4) et présentant un trou de passage (17) en alignement avec le trou de passage (6) du corps (3) ainsi qu'une chambre (19) orientée au moins vers le bas, délimitée par des languettes (8) formées de manière à pouvoir être déplacées élastiquement vers l'axe de cylindre (4) et dans laquelle peut s'enclencher de manière élastique, par le bas, la tête (9) d'une vis pédiculaire (2); et
C) un moyen de serrage (26;91) permettant de déplacer la pince de serrage (7) par rapport au corps (3),
**caractérisé en ce que**
D) le trou de passage (6) ménagé dans le corps (3) est positionné par rapport au trou de passage (17) ménagé dans la pince de serrage (7) de sorte que, lorsque l'on serre le moyen de serrage (26;91), seule la tête d'une vis pédiculaire (2) est bloquée; et
E) une vis de réglage (12) est intégrée dans le moyen de serrage (26;91) de sorte que, lorsque l'on la visse vers le bas, elle bloque un support longitudinal (1) inséré dans le dispositif.

2. Dispositif selon la revendication 1, **caractérisé en ce que** la chambre (19) est réalisée sous forme de cavité en forme de boule creuse.

3. Dispositif selon la revendication 2, **caractérisé en ce que** la pince de serrage (7) présente à l'extérieur, dans la zone de la chambre (19) réalisée sous forme de cavité en forme de boule creuse, une forme conique allant en s'élargissant vers le bas.

4. Dispositif selon l'une des revendications 1 à 3, **caractérisé en ce qu'**une vis de réglage (12) est intégrée dans la pince de serrage (7) ou dans un moyen de serrage (91) de sorte que, lorsque l'on la visse vers le bas, elle bloque le support longitudinal (1).

5. Dispositif selon l'une des revendications 1 à 4, **caractérisé en ce que** la pince de serrage (7) est symétrique à un plan de symétrie.

6. Dispositif selon l'une des revendications 1 à 5, **caractérisé en ce que** le corps (3) est symétrique à un plan de symétrie.

7. Dispositif selon l'une des revendications 1 à 6, **caractérisé en ce que** le corps (3) se subdivise en une partie supérieure (53) et une partie inférieure (52), la surface de séparation s'étendant transversalement à l'axe (4) dans la zone du trou de passage (6) et le trou (25) étant réalisé de manière traversante au moins dans la partie inférieure (52).

8. Dispositif selon l'une des revendications 1 à 7, **caractérisé en ce que** le moyen de serrage (12;56) presse sur le support longitudinal (1) et tire ainsi la pince de serrage (7) dans le corps (3), bloquant ainsi la tête (9) de la vis pédiculaire (2) et le support longitudinal (1).

9. Dispositif selon l'une des revendications 1 à 8, **caractérisé en ce que** la hauteur du trou de passage (6) est dimensionnée de telle sorte que, dans chaque position du moyen de serrage (12;26;56), le support longitudinal (1) repose toujours sur le bord inférieur du trou de passage (6).

10. Dispositif selon l'une des revendications 1 à 9, **caractérisé en ce que** le corps (3) et la pince de serrage (7) sont disposés de manière à empêcher tout mouvement de rotation l'un par rapport à l'autre tout en pouvant cependant coulisser axialement l'un par rapport à l'autre.

11. Dispositif selon l'une des revendications 1 à 10, **caractérisé en ce que** la pince de serrage (7) est disposée dans le corps (3) au moyen d'un ajustement serré.

12. Dispositif selon l'une des revendications 1 à 11, **caractérisé en ce que** le corps (3) présente, à son extrémité inférieure (24), une surface intérieure conique (14) allant en s'élargissant vers le bas et contre laquelle il est possible de faire reposer, de manière coulissante, une surface extérieure conique (18) correspondante formée par les extrémités libres des languettes (8) de la pince de serrage (7).

13. Dispositif selon l'une des revendications 1 à 12, **caractérisé en ce que** la pince de serrage (7) est constituée, dans la zone de contact avec le corps (3), de manière à reposer tangentiellement sur la surface intérieure conique (14) du corps (3), laquelle va en s'élargissant vers le bas.

14. Dispositif selon l'une des revendications 1 à 13, **caractérisé en ce qu'**il comprend en outre une vis pédiculaire (2) pourvue de préférence d'une tête sphérique (9).

15. Dispositif selon la revendication 14, **caractérisé en ce que** la tête (9) de la vis pédiculaire (2) est pourvue d'une structure (15), de préférence en forme de rainures transversales ou de nervures transversales.

16. Dispositif selon la revendication 14 ou 15, **caractérisé en ce que** la tête (9) de la vis pédiculaire (2) présente une forme à six pans creux (16).

17. Dispositif selon l'une des revendications 1 à 16, **caractérisé en ce que** la chambre (19) est réalisée sous forme de cavité en forme de boule creuse, présentant de préférence une forme complémentaire à la forme de la tête (9) de la vis pédiculaire (2) qu'il s'agit de recevoir.

18. Dispositif selon l'une des revendications 1 à 17, **caractérisé en ce que** la chambre (19) est pourvue d'une structure (21), de préférence en forme de rainures transversales ou de nervures transversales.

19. Dispositif selon l'une des revendications 1 à 18, **caractérisé en ce que** le corps (3) est réalisé sous forme de douille en forme de cylindre creux.

20. Dispositif selon la revendication 19, **caractérisé en ce que** le corps (3) est réalisé en une seule pièce.

21. Dispositif selon la revendication 19, **caractérisé en ce que** le corps (3) se compose d'une partie inférieure (52) et d'une partie supérieure (53).

22. Dispositif selon la revendication 21, **caractérisé en ce que** la séparation entre la partie inférieure (52) et la partie supérieure (53) se situe dans la zone du trou de passage (6).

23. Dispositif selon l'une des revendications 1 à 22, **caractérisé en ce que** le trou de passage (6;80;81) du corps (3) est positionné par rapport au trou de passage (17;42;54) de la pince de serrage (7;71;72;73;74;75;76;77) de telle sorte que le support longitudinal (1) repose sur le bord inférieur du trou de passage (6;80;81) du corps (3).

24. Dispositif selon l'une des revendications 1 à 22, **caractérisé en ce que** le trou de passage (6;80;81) du corps (3) est positionné par rapport au trou de passage (17;42;54) de la pince de serrage (7;71;72;73;74;75;76;77) de telle sorte que le support longitudinal (1) repose sur le bord inférieur du trou de passage (17,42;54) de la pince de serrage (7).

25. Dispositif selon l'une des revendications 21 à 24, **caractérisé en ce que** la partie supérieure (53) du corps (3) repose sur le support longitudinal (1).

26. Dispositif selon l'une des revendications 20 à 24, **caractérisé en ce que** le bord supérieur du trou de passage (6,80,81) du corps (3) ne vient pas reposer sur le support longitudinal.

27. Dispositif selon l'une des revendications 1 à 25, **caractérisé en ce que**, en vue de recevoir le support longitudinal (1), un canal (42) ouvert latéralement est ménagé dans la pince de serrage (74;76).

28. Dispositif selon l'une des revendications 1 à 25, **caractérisé en ce que**, en vue de recevoir le support longitudinal (1), un canal (54) ouvert vers le haut est ménagé dans la pince de serrage (72;75;77).

29. Dispositif selon l'une des revendications 1 à 28, **caractérisé en ce que** la pince de serrage (7,75) est pourvue, à l'extrémité supérieure, d'un filetage intérieur (11).

30. Dispositif selon la revendication 29, **caractérisé en ce qu'**une vis de réglage (56) pourvue d'un filetage extérieur (57) est utilisée comme moyen de serrage.

31. Dispositif selon la revendication 30, **caractérisé en ce que** la vis de réglage (56) est pourvue d'un bord (58) présentant un diamètre extérieur qui est de préférence supérieur au diamètre intérieur du corps (3).

32. Dispositif selon la revendication 29, **caractérisé en ce que** le trou de passage (6;80;81) ménagé dans le corps (3) est positionné par rapport au trou de passage (17,42,54) ménagé dans la pince de serrage (7) de sorte que, lorsque l'on serre le moyen de serrage (91), seule la tête sphérique (9) est bloquée.

33. Dispositif selon la revendication 31 ou 32, **caractérisé en ce que** le corps (3) est réalisé en une seule pièce.

34. Dispositif selon la revendication 31 ou 32, **caractérisé en ce que** le corps (3) se compose d'une partie inférieure (52) et d'une partie supérieure (53).

35. Dispositif selon l'une des revendications 31 à 34, **caractérisé en ce que** le trou de passage (6;80;81) ménagé dans le corps (3) est positionné par rapport au trou de passage (17;42;54) ménagé dans la pince de serrage (7;71;72;74;75) de telle sorte que le support longitudinal (1) repose, avant et après le serrage du moyen de serrage (26;91), sur le bord inférieur du trou de passage (6;80;81).

36. Dispositif selon l'une des revendications 31 à 35, **caractérisé en ce que** le trou de passage (6;80;81) ménagé dans le corps (3) est positionné par rapport au trou de passage (17;42;54) ménagé dans la pince de serrage (7;73;76;77) de telle sorte que le support longitudinal (1) repose, même après le serrage du moyen de serrage (26;91), sur le bord inférieur du trou de passage (17;42;54) ménagé dans la pince de serrage (7;73;76;77).

37. Dispositif selon l'une des revendications 31 à 36, **caractérisé en ce que** le trou de passage (80) est ouvert latéralement.

38. Dispositif selon l'une des revendications 31 à 36, **caractérisé en ce que** le trou de passage (81) est ouvert vers le haut.

39. Dispositif selon l'une des revendications 31 à 38, **caractérisé en ce que** le corps (3) est relié au moyen de serrage (26;91) en ce qui concerne la traction et la pression, de préférence par une entaille (94), de telle sorte qu'il est possible de serrer et de desserrer librement le moyen de serrage (26;91) et que, lorsque l'on desserre le moyen de serrage (26;91), le corps (3) est tiré en arrière, libérant ainsi de nouveau la pince de serrage (7;73;76;77).

40. Dispositif selon l'une des revendications 31 à 38, **caractérisé en ce que** la pince de serrage (7;73;76;77) est pourvue, à l'extrémité supérieure, d'un filetage intérieur (11) dans lequel il est possible d'insérer une vis de réglage (12) afin de bloquer, de manière séparée, le support longitudinal (1).

41. Dispositif selon la revendication 36 et selon l'une des revendications 31 à 38, **caractérisé en ce que** le trou de passage (6) est formé ce telle sorte que la partie supérieure (53) ne vient pas reposer sur le support longitudinal (1).

42. Dispositif selon l'une des revendications 1 à 41, **caractérisé en ce que** le trou (25) ménagé dans le corps (3) est réalisé de manière traversante depuis le bas vers le haut.
